# EUROPEAN PATENT SPECIFICATION

(11) **EP 3 435 967 B1**
(45) Date of publication and mention of the grant of the patent: **27.01.2021**
(21) Application number: 17710916.2
(22) Date of filing: 15.03.2017
(51) Int. Cl.: A61K 8/37, A61K 8/86, A61K 8/41, A61Q 5/12

(54) **HAIR CONDITIONING COMPOSITION CONTAINING PENTAERYTHRITOL ESTER AND PPG ALKYL ETHER**
HAARPFLEGEZUSAMMENSETZUNG MIT PENTAERYTHRIT-ESTER UND PPG-ALKYLETHER
COMPOSITION D'APRÈS-SHAMPOING CONTENANT UN/DES ESTERS DE PENTAÉRYTHRITOL ET UN/DES ÉTHERS ALKYLIQUES DE PPG

(30) Priority: 30.03.2016 WO PCT/CN2016/077799
(43) Date of publication of application: 06.02.2019
(73) Proprietor: Beiersdorf AG, 20253 Hamburg (DE)
(72) Inventor: FENG, Ji, Wuhan Hubei 420070 (CN); DONG, Chaochun, Wuhan Hubei 430056 (CN); WANG, Tan, Wuhan Hubei 430056 (CN); BAO, Zhaoxia, Wuhan Hubei 430070 (CN); HONG, Xiaopeng, Wuhan Hubei 430013 (CN)
(86) International application number: PCT/EP2017/056104
(87) International publication number: WO 2017/167579

(56) References cited:
- EP-A2- 2 138 155
- WO-A1-01/08644
- WO-A1-99/13838
- WO-A2-01/74312
- US-A1- 2003 143 174

## Description

### Technical field

The present invention belongs to the cosmetic field and relates to a hair conditioning composition comprising one or more pentaerythritol esters and one or more PPG alkyl ethers. The composition according to the present invention does not weigh down the hair and at the same time reduces the roughness of the hair. Thereby, the composition according to the present invention can be a shampoo, a cleansing or solely a conditioning composition, which may be applied to the hair and afterwards rinsed off with water, or which remains in the dry or wet hair after application.

### Technical background

A beautiful and attractive appearance is a desire for many people. Therefore, an essential part of the daily personal care of humans is focus on the hair of the head. Cleansing and shampoo products are commonly used to remove excess of soil or sebum from the hair. However, these products often leave the hair in a dry and rough condition. To address this issue many cleansing and shampoo products contain conditioning agents to provide a conditioning effect. Alternatively, a separate conditioner product can also be used after cleansing or shampooing the hair. These separate conditioners can be formulated either as a rinse-off product, which is applied to the hair and afterwards rinsed off with water, or as a leave-in product, which remains in the dry or wet hair after application. Generally, separate conditioner provides a far better conditioning effect to the hair than cleansing or shampoo products including conditioning agents.

The purpose of the conditioning agents of the above products is not only to reduce the dryness and roughness of the hair. Moreover, the richness and slipperiness, as well as the gliding and combability of the hair are improved by the conditioning agents. Typically used conditioning agents are silicone containing compounds. However, these are well known for the fact that silicone residues can build up on the hair. As a result these silicone residues weigh down the hair, which reduces the hair volume. In particular, this can become an issue for consumers with fine hair wishing to build up volume. Therefore, it remains desirable to provide alternative conditioning compositions, which provide an enhanced hair volume by reducing the effect of residues weighing down the hair.

EP 2138155 A2 discloses silicone free hair conditioning compositions, which include PPG-3 benzyl ether myristate to reduce the weighing down of hair while providing a conditioning effect. EP 2138155 A2 does not disclose the use of PPG alkyl ether and pentaerythritol ester.

WO 2002100360 A1 teaches the use of a cellulose polymer, a cationic surfactant, a high melting point fatty acid and an aqueous carrier in a hair conditioning composition. PPG-3 myristyl ether and PPG-11 stearyl ether are included in the example formulations as emollient. In the description pentaerythritol ester oils are mentioned as useful components for the formulations. The document is silent on any conditioning effect provided by any PPG alkyl ether and pentaerythritol ester.

WO 0174312 A2 discloses hair conditioning compositions which contain pentaerythritol ester oils (Page 40, line 29 to page 41). These shall provide benefits such as moisturize, smooth feel, manageability control to the hair. WO 0174312 A2 teaches that pentaerythritol ester oils cover the surface of the hair and thereby delivers smoothness and manageability control to the hair. Therefore, it can be assumed that the hair is likely to weigh down by attaching pentaerythritol ester oils to its surface. Moreover, there is no mentioning of PPG alkyl ether in WO 0174312 A2.

Further documents addressing the weigh down effect of hair conditioning compositions are for example WO 0182879 A2, EP 1815841 A1 and EP 2462918 A2. None of the documents cited discloses any guidance to the present invention.

### Summary of invention

The present invention is to provide a conditioning composition which provides an effective conditioning to the hair upon use whereby the effect of hair being weighed down by residues after application of the conditioner is reduced.

It has surprisingly been found out that a hair conditioning composition comprising one or more pentaerythritol esters and one or more PPG alkyl ethers provides not only an effective conditioning of the hair, moreover, it was found that the effect of the hair being weighed down is improved. In particular, it was unexpected that the inventive composition showed a comparable or even an improved conditioning effect relative to silicone oil containing compositions, whereby the effect of the hair being weighed down was found to be reduced in comparison to the silicon oil containing composition.

Especially in the view of the above discussed document WO 0174312 A2 it was surprising that after use of the composition of the present invention the hair does not weigh down.

Accordingly, the first object of the present invention is a conditioning composition for hair comprising one or more pentaerythritol esters of the following formula (I) 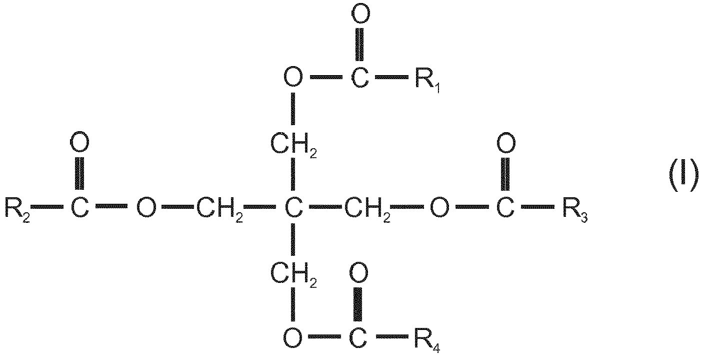 wherein R₁, R₂, R₃ and R₄ are straight or branched, saturated or unsaturated alkyl chains with 5 to 24 C atoms, and one or more PPG alkyl ethers of the following formula (II) 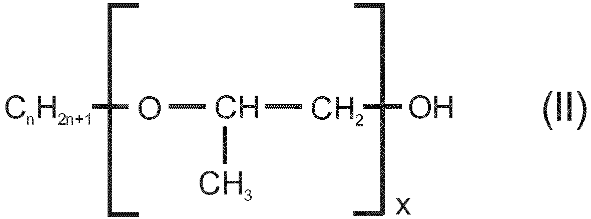 wherein n is an integer in the range from 1 to 12, and x is an integer in the range from 2 to 40, characterized in that the pentaerythritol esters are selected from pentaerythrityl tetrastearate and pentaerythrityl tetraisostearate.

The term "PPG" stands for polypropylene.

A further object of the present invention is the use of the composition of the present invention as conditioner for human hair, whereby the effect of hair being weighed down by the conditioning agents is reduced.

Still a further object of the present invention is the method of conditioning human hair, comprising steps of applying the composition of the present invention to the hair and optionally rinsing off the composition after its application within 1 to 60 minutes.

The most preferred pentaerythritol ester is pentaerythrityl tetraisostearate.

Pentaerythrityl tetraisostearate is commercially available under the trade name CRODAMOL PTIS-LQ-(MV) from the Fa. Croda Inc.

The total concentration of pentaerythritol esters in the composition of the present invention is in the range from 0.05% to 5%, preferably 0.1% to 3% and more preferably 0.15% to 1% by weight calculated to the total composition.

In a preferred embodiment of the present invention only pentaerythrityl tetraisostearate is included as pentaerythritol ester in a concentration in the range from 0.05% to 5%, preferably 0.1% to 3% and more preferably 0.15% to 1% by weight calculated to the total composition.

Conditioning compositions of the present invention comprise one or more PPG alkyl ethers as defined by formula II. Preferred PPG alkyl ethers are defined by n being an integer in the range from 4 to 8 and x being an integer in the range from 2 to 15. More preferred are PPG-14-butyl ether, PPG-3-caprylylether and their mixtures.

PPG-14-butyl ether is commercially available under the trade name UCON Fluid AP from the Fa. Dow Chemical. PPG-3-caprylyl ether is available under the trade name KAO SOFCARE® GP-1 from the Fa. Kao Chemicals.

The total concentration of PPG alkyl ethers in the composition of the present invention is in the range from 0.05% to 5%, preferably 0.1% to 4% and more preferably 0.15 to 2.5% by weight calculated to the total composition.

In a preferred embodiment of the present invention only PPG-14-butyl ether and PPG-3-caprylyl ether are included as PPG alkyl ethers. The total concentration of PPG-14-butyl ether and PPG-3-caprylyl ether is in the range from 0.05% to 5%, preferably 0.1% to 4% and more preferably 0.15 to 2.5% by weight calculated to the total composition. Thereby the weight ratio of PPG-14-butyl ether to PPG-3-caprylyl ether is preferably in the range 2:1 to 1:2, more preferably 1.5:1 to 1:1.5.

The conditioning compositions of the present invention may be a cleansing composition such as a shampoo or cleanser, or a composition which can be applied after cleansing such as a leave-in conditioner or rinse-off conditioner.

The hair conditioning composition of the present invention can further comprise as additional conditioning agent one or more amidoamines defined by the formula (III) 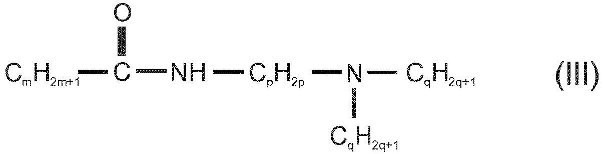 wherein m is an integer in the range from 11 to 23, p is an integer in the range from 1 to 4 and q is an integer in the range from 1 to 4.

By adding one or more amidoamines the volume of the dried hair is further increased after cleansing and/or conditioning the hair with the inventive composition. Preferred amidoamines of the present invention are stearamidopropyl dimethyl amine, stearamidopropyl diethyl amine, stearamidoethyl diethyl amine, stearamidoethyl dimethyl amine, palmitamidopropyl dimethyl amine, palmitamidopropyl diethyl amine, palmitamidoethyl diethyl amine, palmitamidoethyl dimethyl amine, behenamidopropyl dimethyl amine, behenamidopropyl diethyl amine, behenamidoethyl diethyl amine, behenamidoethyl dimethyl amine, arachidamidopropyl dimethyl amine, arachidamidopropyl diethyl amine, arachidamidoethyl diethyl amine, arachidamidoethyl dimethyl amine and mixtures thereof. The most preferred amidoamines are stearamidopropyl dimethyl amine and/or behenamidopropyl dimethyl amine. According to the invention amidoamines as defined above are not considered as surfactant.

Stearamidopropyl dimethyl amine is commercially available under the trade name TEGO AMID S 18 from the Fa. Evonik Industries. Behenamidopropyl dimethyl amine can commercially be obtained under the trade name AMIDET® APA-22 from the Fa. Kao Chemicals.

The concentration of the one or more amidoamines in total is in the range of preferably 0.1% to 5%, more preferably 0.25% to 4% and most preferably 0.4% to 3% by weight calculated to the total composition.

In a preferred embodiment of the present invention only stearamidopropyl dimethyl amine and/or behenamidopropyl dimethyl amine are included as amidoamines. In this embodiment of the present invention the total concentration of stearamidopropyl dimethyl amine and/or behenamidopropyl dimethyl amine is in the range of preferably 0.1% to 5%, more preferably 0.25% to 4% and most preferably 0.4% to 3% by weight calculated to the total composition.

The composition of the present invention can preferably contain one or more fatty alcohols. Preferably the fatty alcohols correspond to the formula R₅-OH in which R₅ stands for a saturated or unsaturated, linear or branched hydrocarbon group, wherein the hydrocarbon group comprises 8 to 30 carbon atoms, more preferably 14 to 18 carbon atoms. Among those preferred fatty alcohols linear, saturated alcohols such as cetyl alcohol, stearyl alcohol and/or mixtures thereof are the most preferred ones.

Cetyl alcohol is commercially available under the trade name Lanette® 16 from the Fa. BASF. Similarly, stearyl alcohol can be purchased under the trade name Lanette® 18 from the Fa. BASF. Mixtures of Cetyl alcohol and stearyl alcohol can also be obtained from the Fa. BASF. The trade name of the mixture is called Lanette® O.

The fatty alcohols are preferably used in a total concentration of 2% to 12% by weight, more preferably 3% to 10% by weight, most preferably 4% to 9% by weight calculated to the total composition.

In a preferred embodiment of the present invention only cetyl alcohol, stearyl alcohol and/or mixtures thereof are included as fatty alcohol. In this embodiment of the present invention the concentration of cetyl alcohol, stearyl alcohol and/or mixtures thereof is in the range of 2% to 12% by weight, preferably 3% to 10% by weight, more preferably 4% to 9% by weight calculated to the total composition.

The compositions according to the invention may also preferably comprise one or more cationic surfactants. Suitable cationic surfactants which can be used in the conditioning compositions of the present invention are quaternary ammonium salts defined by the formula (IV) 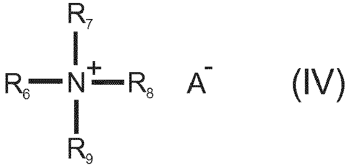
wherein R₆ and R₇ stand for a hydrogen atom or an alkyl chain of 1 to 4 carbon atoms which are optionally substituted with one or more hydroxyl groups,
and wherein R₈ stands for a saturated or unsaturated, branched or non-branched alky chain of 8 to 24 carbon atoms,
or R₈ stands for R₁₀CON(CH₂)_{y} group, wherein R₁₀ is a saturated or unsaturated, branched or non-branched alkyl chain with 7 to 21 carbon atoms and y is an integer in the range from 1 to 4,
or R₈ stands for R₁₁COO(CH₂)_{z} group, wherein R₁₁ stands for a saturated or unsaturated, branched or non-branched alkyl chain with 7 to 21 carbon atoms and z is an integer in the range from 1 to 4,
and wherein R₉ stands for a hydrogen atom or for a saturated or unsaturated, branched or non-branched alky chain of 8 to 24 carbon atoms,
or R₉ stands for a R₁₀CON(CH₂)_{y} or R₁₁COO(CH₂)_{z} group, wherein R₁₀, R₁₁, y and z have the same meaning as above,
and A is an anion, preferably chloride or bromide.

Suitable but not limiting examples of above defined cationic surfactants are octyltrimethylammonium chloride, decyltrimethylammonium chloride, dodecyltrimethylammonium chloride, cetyltrimethylammonium chloride, stearyltrimethylammonium chloride, behenyltrimethylammonium chloride, tetramethylammonium chloride, tetraethylammonium chloride, cetylpyridinium chloride, octyldimethylbenzylammonium chloride, decyldimethylbenzylammonium chloride, stearyldimethylbenzylammonium chloride, didodecyldimethylammonium chloride and dioctadecyldimethylammonium chloride.

The most preferred cationic surfactants to be contained in the compositions of the present invention are cetyltrimethylammonium chloride and/or behenyltrimethylammonium chloride. Cetyltrimethylammonium chloride is available under the trade name VARISOFT 300 from the Fa. Evonik Industries or Dehyquart® A-CA from the Fa. BASF. Behenyltrimethylammonium chloride is commercially available under the trade name QUARTAMIN® AB from the Fa. Kao Chemicals.

The cationic surfactants are preferably used in a concentration in the range from 0.1% to 5% by weight, more preferably 0.2% to 3% by weight, calculated to the total composition. The values are referring to the active content of the cationic surfactants.

In a preferred embodiment of the present invention only cetyltrimethylammonium chloride and/or behenyltrimethylammonium chloride are included as cationic surfactant. In this embodiment of the present invention the total concentration of cetyltrimethylammonium chloride and/or behenyltrimethylammonium chloride is preferably in the range from 0.1% to 5% by weight, more preferably 0.2% to 3% by weight, calculated to the total composition. The values are referring to the active content of the cationic surfactants.

Additionally citric acid or lactic acid may be added to the composition of the present invention to adjust the pH value. A pH value in the range from 3.0 to 6.9, preferably 3.5 to 6.5 is desirable for hair conditioning compositions.

Additionally, the hair conditioning composition of the present invention may comprise water in a concentration of preferably more than 80% by weight, more preferably more than 85% by weight calculated to the total weight of the composition.

Furthermore, the hair conditioning composition of the present invention may preferably comprise one or more surfactants selected from anionic, amphoteric and/or nonionic surfactants.

Suitable anionic surfactants include alkyl sulphates, alkyl ether sulphates, alkaryl sulphonates, alkanoyl isethionates, alkyl succinates, alkyl sulphosuccinates, alkyl ether sulphosuccinates, alkyl phosphates, alky ether phosphates, alkyl ether carboxylic acids and salts thereof, e.g. the sodium, magnesium, ammonium and mono-, di- and triethanolamine salts, or suitable mixtures thereof. The alkyl groups of the above listed anionic surfactants contain from 8 to 18 carbon atoms wherein the alkyl groups can be saturated or unsaturated. The alkyl ether sulphates, alkyl ether sulphosuccinates, alky ether phosphates, alkyl ether carboxylic acids and salts thereof contain from 1 to 20 ethylene oxide or propylene oxide units per molecule.

Preferred anionic surfactants for use in the conditioning composition of the present invention are sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, and/or lauryl ether carboxylic acid.

Typical examples of amphoteric surfactants, which can be contained in the conditioning composition of the present invention, are alkyl betaines, alkyl amidopropyl betaines, cocoamidopropylbetaine, alkyl sulphobetaines, alkyl amine oxides, alkyl amphoacetates, alkyl amphopropionates, alkyl amidopropyl hydroxysultaines and/or acyl taurates, wherein the alkyl and acyl groups comprise 8 to 19 carbon atoms. Preferred amphoteric surfactants are selected from lauryl amine oxide, cocodimethyl sulphopropyl betaine, lauryl betaine and cocamindopropylbetaine, sodium lauroamphoacetate and/or sodium cocoamphoacetate.

Suitable non-ionic surfactants include alkylamine oxide, N-alkyl-N-methyl glucamide, alkyl polyglucosides, coco mono- or diethanolamide, coco mono-isopropanolamide and/or condensation products of aliphatic (Cs to C₁₈) primary, secondary linear or branched chain alcohols or phenols with ethylene oxides, generally having from 2 to 30 ethylene oxide groups.

Compositions of the present invention may further contain one or more silicone oils as conditioning agent. Typically used silicone oils are dimethicone, dimethiconol, polydimethylsiloxane and/or their derivatives. However, as the composition of the present invention solely provides a conditioning of the hair, it is preferred that the composition of the present invention is free of silicone oils. Free of silicone oils means that the total concentration of silicone oils is less than 0.001% by weight calculated to the total composition.

Hence, a further advantage of the present invention is that hair conditioning compositions can preferably be formulated without the use of silicone oils while maintaining their conditioning properties.

The compositions of the present invention may further contain cationic polymers known under CTFA category name polyquaternium-j as additional conditioning agent, wherein the letter j is an integer in the range from 1 to 100. Preferred polyquaternium polymers, which are known for their hair conditioning properties, are polyquaternium-7, polyquaternium-11, polyquaternium-44, polyquaternium-70 and polyquaternium-74.

However, as the composition of the present invention solely provides a conditioning of the hair, it is preferred that the composition of the present invention is free from polyquaternium polymers. The term "free from" means in the sense of the present invention that the total concentration of one or more polyquaternium polymers in the composition of the present invention does not exceed 0.05% by weight, calculated to the total composition.

Further cationic polymers which may be applied as additional conditioning agent in the composition of the present invention are for example the silicone-free quaternium polymers known under the CTFA category name quaternium-k, wherein the letter k is an integer in the range from 8 to 95.

However, as the composition of the present invention solely provides a conditioning of the hair, it is preferred that the composition of the present invention is free from quaternium polymers. The term "free from" means in the sense of the present invention that the total concentration of one or more quaternium polymers in the composition of the present invention does not exceed 0.05% by weight, calculated to the total composition.

The composition of the present invention may further comprise one or more cellulose containing ionic or nonionic polymers, which provide an additional conditioning effect. These are for example cellulose gum, cocodimonium hydroxypropyloxyethylcellulose, ethyl cellulose, guar gum, guar hydroxytrimonium chloride, guar hydroxypropyltrimonium chloride, hydroxybutyl cellulose, cetyl hydroxyethyl cellulose, hydroxyethyl cellulose, hydroxyethyl ethylcellulose, hydroxymethyl cellulose, hydroxypropyl cellulose, hydroxypropyl guar, hydroxypropyl guar hydroxypropyltrimonium chloride.

However, as the composition of the present invention solely provides a conditioning of the hair, it is preferred that the composition of the present invention is free from cellulose containing ionic or nonionic polymers. The term "free from" means in this context that the total concentration of one or more cellulose containing ionic or nonionic polymers in the composition of the present invention does not exceed 0.05% by weight calculated to the total composition.

Another category of conditioning agents which may optionally be added to the composition of the present invention are protein and polyprotein hydrolysates, such as hydrolyzed silk, hydrolyzed wheat protein and/or hydrolyzed collagen.

The conditioning composition of the present invention may further comprise one or more preservatives. Suitable preservatives to be comprised in the composition of the present invention are for example phenoxyethanol, ethylhexyglycerin methylparaben, ethylparaben, benzyl alcohol, methylisothiazolinone, methylchloroisothiazolinone and/or DMDM hydantoin.

Furthermore, the composition of the present invention is preferably free from esters of PPG alkyl ethers or PPG aryl ethers. The term "free from" means in this context that the total concentration of one or more esters of PPG alkyl ethers or PPG aryl ethers in the composition of the present invention does not exceed 0.05% by weight, calculated to the total composition.

The conditioning composition of the present invention may further comprise pearlescent pigments, mica and/or effect pigments to prepare visually attractive compositions.

The composition according to the invention may also comprise substances commonly contained in hair conditioning compositions. These are for example UV-filter, dyes, ubiquinone such as coenzyme Q10, vitamins, plant extracts, antioxidants such as oryzanol, and fragrances.

It is further advantageous if the inventive composition is filled and kept in a jar, a bottle, a squeeze bottle, a pump spray or an aerosol can.

### Examples

The following examples should illustrate the compositions of this invention, without, however, intending to limit the invention to these examples. The numerical values in the examples are percentages by weight, based on the total weight of the preparations. The compositions are prepared by mixing the individual components in water.

### Examples 1-8: Rinse-off conditioner

| **Type: Rinse-off Conditioner** | Ex.1 | Ex.2 | Ex.3 | Ex.4 | Ex.5 | Ex.6 | Ex.7 | Ex.8 |
|---|---|---|---|---|---|---|---|---|
| Ingredient | Com. | Com. | Com. | Com. | Com. | Com. | Inv. | Inv. |
| Water | ad 100 | ad 100 | ad 100 | ad 100 | ad 100 | ad 100 | ad 100 | ad 100 |
| Cetrimonium Chloride¹ | 1.5 | 1.5 | 1.5 | 1.5 | 1.5 | 1.5 | 1.5 | 1.5 |
| Stearamidopropyl Dimethylamine ² | 1.5 | 1.5 | 1.5 | 1.5 | 1.5 | 1.5 | 1.5 | 1.5 |
| Lactic Acid | 0.45 | 0.45 | 0.45 | 0.45 | 0.45 | 0.45 | 0.45 | 0.45 |
| Stearyl Alcohol | 4 | 4 | 4 | 4 | 4 | 4 | 4 | 4 |
| Cetyl Alcohol | 2 | 2 | 2 | 2 | 2 | 2 | 2 | 2 |
| Dimethicone ³ | 0.5 | 1 | - | - | - | - | - | - |
| Pentaerythrityl Tetraisostearate ⁴ | - | - | - | 0.2 | - | - | 0.2 | 0.2 |
| PPG-14 Butyl Ether ⁵ | - | - | - | - | 0.2 | - | - | 0.2 |
| PPG-3 Caprylyl Ether ⁶ | - | - | - | - | - | 0.2 | 0.2 | 0.2 |
| Fragrance | 0.25 | 0.25 | 0.25 | 0.25 | 0.25 | 0.25 | 0.25 | 0.25 |
| Preservatives, pH adjustment | Up to 2.0% | Up to 2.0% | Up to 2.0% | Up to 2.0% | Up to 2.0% | Up to 2.0% | Up to 2.0% | Up to 2.0% |

| | | | | | | | | |
|---|---|---|---|---|---|---|---|---|
| Com = Comparative example, not according to the invention Inv = Inventive example, according to the invention ¹ VARISOFT 300, from Evonik ² TEGO AMID S 18, from Evonik ³ Xiameter PMX-200 Fluid, from Dow Corning ⁴ CRODAMOL PTIS-LQ-(MV), from Croda ⁵ UCON Fluid AP, from Dow ⁶ Sofcare GP-1, from Kao | | | | | | | | |

The following test was carried out to show that by use of the inventive composition a solution for the problem introduced above is provided. The test performed was a half head test. That means a test was performed wherein the hair of one side of the head was treated with one composition while the hair on the other side of the head was treated with another composition. The assessment was carried out by trained hairdressers. Asian black hair was used for the test presented herein.

The conditioning of hair is dependent on a number of parameters. According to the invention the conditioning of hair corresponds to the reduction of roughness, as well as an enhancement of the richness, slipperiness, glide ability and combability of the hair. Therefore, in order to value the conditioning effect of the compositions all the following parameters were assessed by the hair dressers: Richness, Slipperiness, gliding, combability and anti-roughness.

The detailed test procedure was as follows:
Initially, the condition of the hair of each subject was evaluated by the hair dresser. Then the hair of each subject was washed using a shampoo composition which was free of any conditioning agent. For that purpose the hair was wetted with warm water, then shampoo was applied and spread through the hair for 15 seconds. For each test the same amount of shampoo was applied. Afterwards the shampoo composition was rinsed off the hair with warm water until no shampoo remained. Before applying the example compositions to be tested, the hair was separated from the middle of the head and each side clipped up.

For each test, the clip on one side of the head was released and 1 g of one of the example compositions **1** to **8** per 10 g hair was spread on the wet hair for 15 seconds. Thereby the richness and slipperiness was accessed by the hair dresser. Each composition remained in the hair for 3 to 5 minutes.

Afterwards, the example composition was rinsed-off with water until the hair was visibly free of the applied composition. Thereby, the gliding while rinsing was accessed by the hair dresser.

After rinsing, the spare water was removed from the hair using a towel. Subsequently, the wet combability was accessed by the hair dresser using a fine-toothed comb. Then the wet gliding was evaluated by the hair dresser.

To access the remaining parameter the hair was fully blow-dried. Then the combability of dry hair was accessed by the hair dresser using a fine-toothed comb. Furthermore, the gliding of the dry hair, the roughness on the tip of the hair and the question if the hair does not weigh down were evaluated by the hair dresser.

Each parameter was rated with a score value between 1 and 10, whereby 1 means bad and 10 corresponds to good. Here, it should be noted that the roughness was assessed as anti-roughness parameter to ensure that a score of 10 corresponds to a positive conditioning effect. The same is true for the analysis of the weigh down effect. In the case the hair does not weigh down at all a score of 10 is given.

As the parameters richness, slipperiness, gliding, combability and anti-roughness contribute to the conditioning capabilities of the compositions, the scores achieved for the parameters were summed to value the total conditioning performance of the compositions.

All the results are presented in the following Table:

| **Salon test** | **Ex.1** | **Ex.2** | **Ex.3** | **Ex.4** | **Ex.5** | **Ex.6** | **Ex.7** | **Ex.8** |
|---|---|---|---|---|---|---|---|---|
| Richness (spread) | 7.13 | 7.13 | 6.2 | 6.5 | 6.65 | 6.3 | 7.03 | 7.5 |
| Slipperiness (spread) | 7.3 | 7.38 | 6.05 | 6.85 | 6.55 | 6.3 | 7.05 | 7.63 |
| Gliding (rinsing) | 8.5 | 9 | 7.05 | 7.85 | 7.55 | 7.25 | 8.5 | 9 |
| Combability (wet hair) | 7.5 | 8.5 | 6 | 6.8 | 6.4 | 6.3 | 7.53 | 8.63 |
| Gliding (wet hair) | 6.85 | 7.63 | 5.75 | 6.65 | 6.2 | 5.75 | 7.35 | 7.55 |
| Combability (dry hair) | 8 | 8.63 | 7.05 | 7.25 | 7.4 | 7.55 | 8.13 | 8.63 |
| Gliding (dry hair) | 7 | 7.5 | 6.8 | 7.3 | 7.53 | 7.5 | 7.43 | 7.75 |
| Anti-roughness (dry hair) | 8.75 | 9.38 | 8.25 | 8.4 | 8.4 | 8.6 | 8.75 | 9.38 |
| Conditioning value | 61.03 | 65.15 | 53.15 | 57.6 | 56.68 | 55.55 | 61.77 | 66.07 |
| Does not weigh down your hair | 6.9 | 6.75 | 7.2 | 7.15 | 7.15 | 7.25 | 7.05 | 7 |

In the above Table showing the results of the tests the lowest conditioning value was determined for the reference composition 3, which did neither contain a pentaerythritol ester nor a PPG alkyl ether. Considering the differences between the compositions containing only one of the inventive substances in the samples 4, 5 and 6 with respect to the composition value determined for the reference composition 3, an over additive conditioning value was determined for the inventive compositions 7 and 8.

Comparing the conditioning values achieved with the silicon containing composition 1 to the inventive composition 7, it has to be noted that a comparable or better condition value is achieved using the inventive composition. The same is true for the comparison of the results achieved for the compositions 2 and 8. Here, it needs to be emphasized that the total concentration of the pentaerythritol ester and the PPG alkyl ether in the inventive composition 7 is lower than the total concentration of silicone oil in the composition 1. The same is true for the inventive composition 8 and the silicone oil containing composition 2.

The results for the question, if after the conditioning the hair is weighed down, reveal that the inventive composition outperforms silicon containing compositions. The effect of the conditioner residues weighing the hair down has been found to be reduced for the use of the inventive composition.

## Claims

1. Hair conditioning composition comprising
a. one or more pentaerythritol esters of the following formula (I) 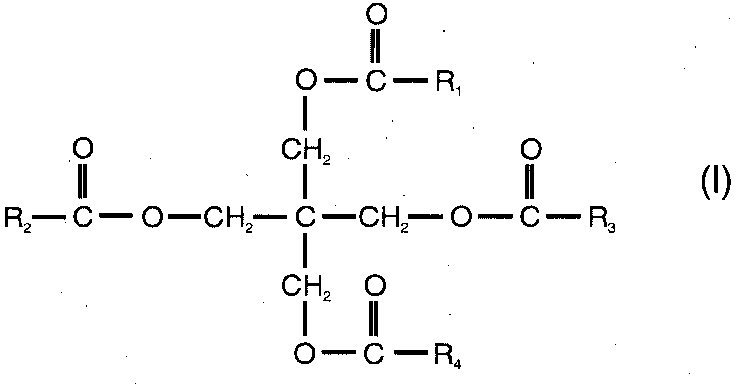 wherein R₁, R₂, R₃ and R4 are straight or branched, saturated or unsaturated alkyl chains with 5 to 24 C atoms, and
b. and one or more PPG alkyl ethers of the following formula (II) 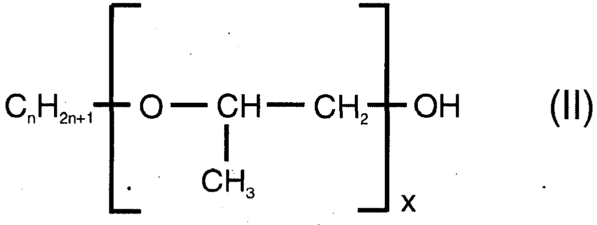 wherein n is an integer in the range from 1 to 12, and x is an integer in the range from 2 to 40;
**characterized in that** the pentaerythritol esters are selected from pentaerythrityl tetrastearate and pentaerythrityl tetraisostearate.

2. Composition according to claim 1 **characterized in that** the total concentration of the one or more pentaerythritol esters is in the range from 0.05% to 5%, preferably 0.1% to 3% and more preferably 0.15% to 1% by weight calculated to the total composition.

3. Composition according to any of the claims 1 to 2 **characterized in that** the PPG alkyl ethers are **characterized by** the following formula (II) 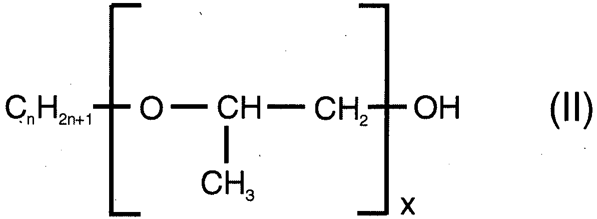 wherein n is an integer in the range from 4 to 8 and x is an integer in the range from 2 to 15.

4. Composition according to any of the claims 1 to 3 **characterized in that** the total concentration of the one or more PPG alkyl ethers is in the range from 0.05% to 5%, preferably 0.1% to 4% and more preferably 0.15 to 2.5% by weight calculated to the total composition.

5. Composition according to any of the claims 1 to 4 **characterized in that** the composition further comprises one or more amidoamines defined by the formula (III) 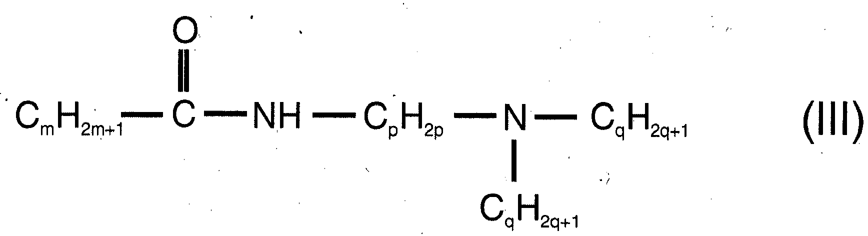 wherein m is an integer in the range from 11 to 23, p is an integer in the range from 1 to 4 and q is an integer in the range from 1 to 4.

6. Composition according to claim 5 **characterized in that** the one or more amidoamines are selected from stearamidopropyl dimethyl amine, stearamidopropyl diethyl amine, stearamidoethyl diethyl amine, stearamidoethyl dimethyl amine, palmitamidopropyl dimethyl amine, palmitamidopropyl diethyl amine, palmitamidoethyl diethyl amine, palmitamidoethyl dimethyl amine, behenamidopropyl dimethyl amine, behenamidopropyl diethyl amine, behenamidoethyl diethyl amine, behenamidoethyl dimethyl amine, arachidamidopropyl dimethyl amine, arachidamidopropyl diethyl amine, arachidamidoethyl diethyl amine, arachidamidoethyl dimethyl amine and mixtures thereof.

7. Composition according to any of the claims 5 to 6 **characterized in that** the concentration of the one or more amidoamines in total is in the range of 0.1% to 5%, preferably 0.25% to 4% and more preferably 0.4% to 3% by weight calculated to the total composition.

8. Composition according to any of the claims 1 to 7 **characterized in that** the composition further comprises one or more fatty alcohols of the formula R₅-OH in which R₅ stands for a saturated or unsaturated, linear or branched hydrocarbon group, wherein the hydrocarbon group comprises 8 to 30 carbon atoms.

9. Composition according to any of the claims 1 to 8 **characterized in that** the composition further comprises one or more quaternary ammonium salts defined by the formula (IV) 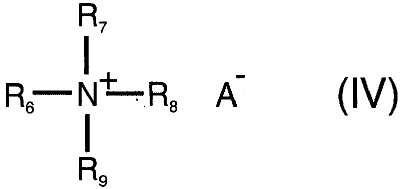
wherein R₆ and R₇ stand for a hydrogen atom or an alkyl chain of 1 to 4 carbon atoms which are optionally substituted with one or more hydroxyl groups,
and wherein R₈ stands for a saturated or unsaturated, branched or non-branched alky chain of 8 to 24 carbon atoms,
or R₈ stands for R₁₀CON(CH₂)_{y} group, wherein R₁₀ is a saturated or unsaturated, branched or non-branched alkyl chain with 7 to 21 carbon atoms and y is an integer in the range from 1 to 4,
or R₈ stands for R₁₁COO(CH₂)_{z} group, wherein R₁₁ stands for a saturated or unsaturated, branched or non-branched alkyl chain with 7 to 21 carbon atoms and z is an integer in the range from 1 to 4,
and wherein Rg stands for a hydrogen atom or for a saturated or unsaturated, branched or non-branched alky chain of 8 to 24 carbon atoms,
or R₉ stands for a R₁₀CON(CH₂)_{y} or R₁₁COO(CH₂)_{z} group, wherein R₁₀, R₁₁, y and z have the same meaning as above, and
A is an anion, preferably chloride or bromide.

10. Composition according to any of the claims 1 to 9 **characterized in that** the composition further comprises water in a concentration of more than 80% by weight calculated to the total weight of the composition.

11. Composition according to any of the claims 1 to 10 **characterized in that** the composition is free of silicone oils.

12. Composition according to any of the claims 1 to 11 **characterized in that** the composition is free from cellulose containing ionic or nonionic polymers.

13. Composition according to any of the claims 1 to 12 **characterized in that** the composition is free from polyquaternium or quaternium polymers.

14. Composition according to any of the claims 1 to 13 **characterized in that** the composition further comprises one or more cationic, anionic, amphoteric and/or nonionic surfactants.

15. Method of conditioning human hair, comprising steps of applying the composition according to any of claims 1 to 14 the hair and optionally rinsing off the composition after its application within 1 to 60 minutes.

## Patentansprüche

1. Haarkonditionierungszusammensetzung, umfassend
a. einen oder mehrere Pentaerythritolester der folgenden Formel (I) 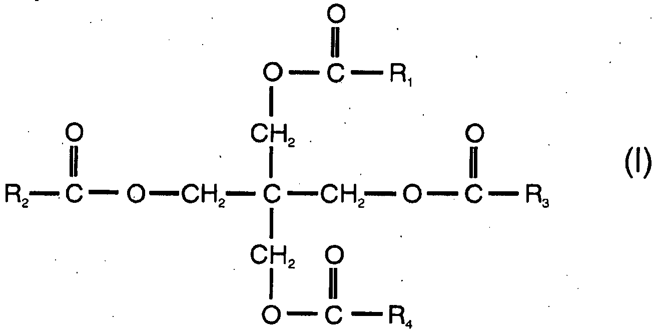 wobei R₁, R₂, R₃ und R₄ für gerade oder verzweigte, gesättigte oder ungesättigte Alkylketten mit 5 bis 24 C-Atomen stehen, und
b. und einen oder mehrere PPG-Alkylether der folgenden Formel (II) 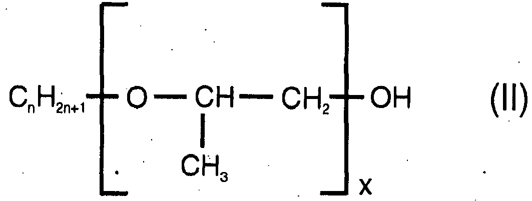
wobei n für eine ganze Zahl im Bereich von 1 bis 12 steht und x für eine ganze Zahl im Bereich von 2 bis 40 steht;
**dadurch gekennzeichnet, dass** die Pentaerythritolester aus Pentaerythrityltetrastearat und Pentaerythrityltetraisostearat ausgewählt sind.

2. Zusammensetzung nach Anspruch 1, **dadurch gekennzeichnet, dass** die Gesamtkonzentration des einen bzw. der mehreren Pentaerythritolester im Bereich von 0,05 bis 5 Gew.-%, vorzugsweise 0,1 bis 3 Gew.-% und weiter bevorzugt 0,15 bis 1 Gew.-%, berechnet auf die gesamte Zusammensetzung, liegt.

3. Zusammensetzung nach einem der Ansprüche 1 bis 2, **dadurch gekennzeichnet, dass** die PPG-Alkylether durch die folgende Formel (II) gekennzeichnet sind: 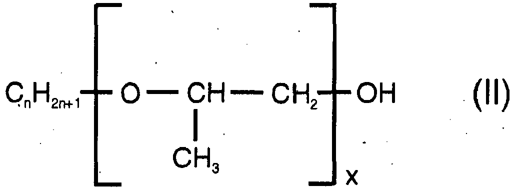 wobei n für eine ganze Zahl im Bereich von 4 bis 8 steht und x für eine ganze Zahl im Bereich von 2 bis 15 steht.

4. Zusammensetzung nach einem der Ansprüche 1 bis 3, **dadurch gekennzeichnet, dass** die Gesamtkonzentration des einen bzw. der mehreren PPG-Alkylether im Bereich von 0,05 bis 5 Gew.-%, vorzugsweise 0,1 bis 4 Gew.-% und weiter bevorzugt 0,15 bis 2,5 Gew.-%, berechnet auf die gesamte Zusammensetzung, liegt.

5. Zusammensetzung nach einem der Ansprüche 1 bis 4, **dadurch gekennzeichnet, dass** die Zusammensetzung ferner ein oder mehrere Amidoamine gemäß der Formel (III) umfasst: 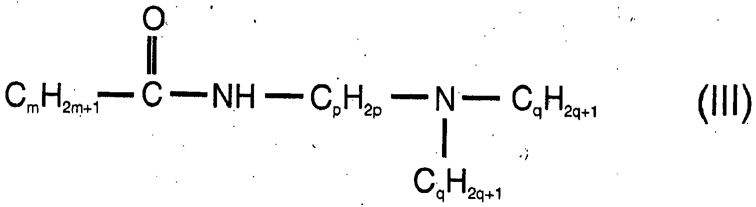 wobei m für eine ganze Zahl im Bereich von 11 bis 23 steht, p für eine ganze Zahl im Bereich von 1 bis 4 steht und q für eine ganze Zahl im Bereich von 1 bis 4 steht.

6. Zusammensetzung nach Anspruch 5, **dadurch gekennzeichnet, dass** das eine bzw. die mehreren Amidoamine aus Stearamidopropyldimethylamin, Stearamidopropyldiethylamin, Stearamidoethyldiethylamin, Stearamidoethyldimethylamin, Palmitamidopropyldimethylamin, Palmitamidopropyldiethylamin, Palmitamidoethyldiethylamin, Palmitamidoethyldimethylamin, Behenamidopropyldimethylamin, Behenamidopropyldiethylamin, Behenamidoethyldiethylamin, Behenamidoethyldimethylamin, Arachidamidopropyldimethylamin, Arachidamidopropyldiethylamin, Arachidamidoethyldiethylamin, Arachidamidoethyldimethylamin und Mischungen davon ausgewählt sind.

7. Zusammensetzung nach einem der Ansprüche 5 bis 6, **dadurch gekennzeichnet, dass** die Konzentration des einen bzw. der mehreren Amidoamine insgesamt im Bereich von 0,1 bis 5 Gew.-%, vorzugsweise 0,25 bis 4 Gew.-% und weiter bevorzugt 0,4 bis 3 Gew.-%, berechnet auf die gesamte Zusammensetzung, liegt.

8. Zusammensetzung nach einem der Ansprüche 1 bis 7, **dadurch gekennzeichnet, dass** die Zusammensetzung ferner einen oder mehrere Fettalkohole der Formel R₅-OH umfasst, wobei R₅ für eine gesättigte oder ungesättigte, lineare oder verzweigte Kohlenwasserstoffgruppe steht, wobei die Kohlenwasserstoffgruppe 8 bis 30 Kohlenstoffatome umfasst.

9. Zusammensetzung nach einem der Ansprüche 1 bis 8, **dadurch gekennzeichnet, dass** die Zusammensetzung ferner ein oder mehrere quaternäre Ammoniumsalze gemäß der Formel (IV) umfasst: 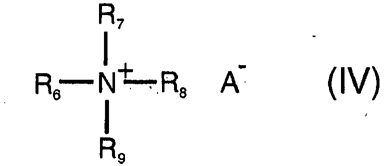
wobei R₆ und R₇ für ein Wasserstoffatom oder eine Alkylgruppe mit 1 bis 4 Kohlenstoffatomen, die gegebenenfalls durch eine oder mehrere Hydroxylgruppen substituiert ist, stehen
und wobei R₈ für eine gesättigte oder ungesättigte, verzweigte oder unverzweigte Alkylkette mit 8 bis 24 Kohlenstoffatomen steht
oder R₈ für eine R₁₀CON(CH₂)_{y}-Gruppe steht, wobei R₁₀ für eine gesättigte oder ungesättigte, verzweigte oder unverzweigte Alkylkette mit 7 bis 21 Kohlenstoffatomen steht und y für eine ganze Zahl im Bereich von 1 bis 4 steht,
oder R₈ für eine R₁₁COO(CH₂)_{z}-Gruppe steht, wobei R₁₁ für eine gesättigte oder ungesättigte, verzweigte oder unverzweigte Alkylkette mit 7 bis 21 Kohlenstoffatomen steht und z für eine ganze Zahl im Bereich von 1 bis 4 steht,
und wobei R₉ für ein Wasserstoffatom für eine gesättigte oder ungesättigte, verzweigte oder unverzweigte Alkylkette mit 8 bis 24 Kohlenstoffatomen steht
oder R₉ für eine R₁₀CON(CH₂)_{y}-Gruppe oder R₁₁COO(CH₂)_{z}-Gruppe steht, wobei R₁₀, R₁₁, y und z die gleiche Bedeutung wie oben haben,
und A für ein Anion, vorzugsweise Chlorid oder Bromid, steht.

10. Zusammensetzung nach einem der Ansprüche 1 bis 9, **dadurch gekennzeichnet, dass** die Zusammensetzung ferner Wasser in einer Konzentration von mehr als 80 Gew.-%, berechnet auf das Gesamtgewicht der Zusammensetzung, umfasst.

11. Zusammensetzung nach einem der Ansprüche 1 bis 10, **dadurch gekennzeichnet, dass** die Zusammensetzung frei von Silikonölen ist.

12. Zusammensetzung nach einem der Ansprüche 1 bis 11, **dadurch gekennzeichnet, dass** die Zusammensetzung frei von cellulosehaltigen ionischen oder nichtionischen Polymeren ist.

13. Zusammensetzung nach einem der Ansprüche 1 bis 12, **dadurch gekennzeichnet, dass** die Zusammensetzung frei von Polyquaternium- oder Quaternium-Polymeren ist.

14. Zusammensetzung nach einem der Ansprüche 1 bis 13, **dadurch gekennzeichnet, dass** die Zusammensetzung ferner ein oder mehrere kationische, anionische, amphotere und/oder nichtionische Tenside umfasst.

15. Verfahren zum Konditionieren von menschlichem Haar, umfassend die Schritte des Aufbringens der Zusammensetzung nach einem der Ansprüche 1 bis 14 auf das Haar und gegebenenfalls des Abspülens der Zusammensetzung nach dem Aufbringen innerhalb von 1 bis 60 Minuten.

## Revendications

1. Composition de conditionnement capillaire comprenant
a. un ou plusieurs esters de pentaérythritol de la formule (I) suivante 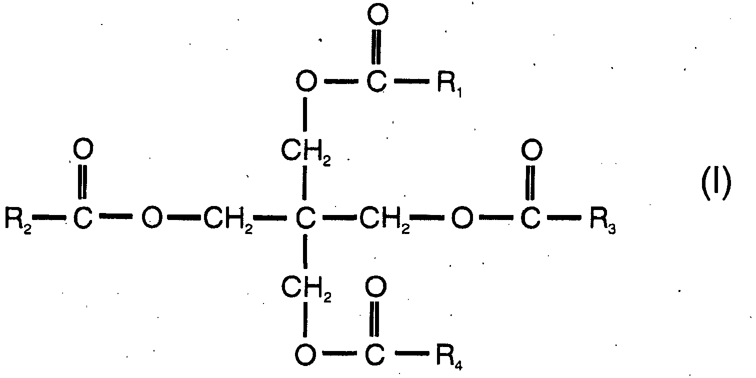 dans laquelle R₁, R₂, R₃ et R₄ sont des chaînes alkyle linéaires ou ramifiées, saturées ou insaturées avec 5 à 24 atomes C, et
b. un ou plusieurs éthers d'alkyle de PPG de la formule (II) suivante 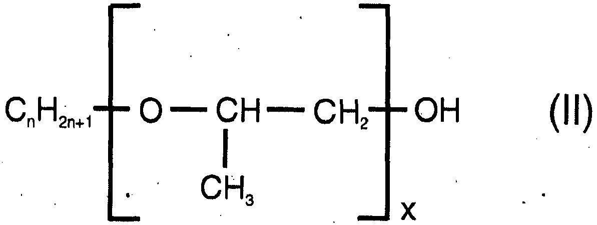
dans laquelle n est un entier dans la plage de 1 à 12, et x est un entier dans la plage de 2 à 40 ;
**caractérisé en ce que** les esters de pentaérythritol sont choisis parmi le tétrastéarate de pentaérythrityle et le tétraisostéarate de pentaérythryle.

2. Composition selon la revendication 1 **caractérisée en ce que** la concentration totale des un ou plusieurs esters de pentaérythritol est dans la plage de 0,05 % à 5%,
de préférence 0,1 % à 3 % et plus préférablement 0,15 % à 1 % calculé par rapport à la composition totale.

3. Composition selon l'une quelconque des revendications 1 à 2 **caractérisée en ce que** les éthers d'alkyle de PPG sont **caractérisés par** la formule (II) suivante 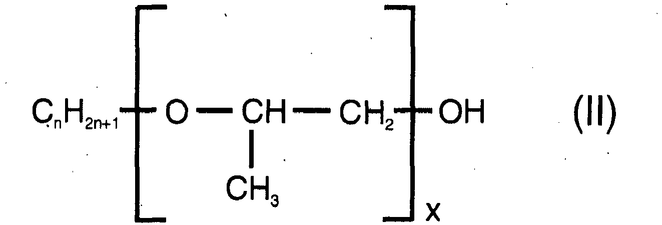 dans laquelle n est un entier dans la plage de 4 à 8 et x est un entier dans la plage de 2 à 15.

4. Composition selon l'une quelconque des revendications 1 à 3 **caractérisée en ce que** la concentration totale des un ou plusieurs éthers d'alkyle de PPG est dans la plage de 0,05 % à 5 %, de préférence 0,1 % à 4 % et plus préférablement 0,15 à 2,5 % en poids calculée par rapport à la composition totale.

5. Composition selon l'une quelconque des revendications 1 à 4 **caractérisée en ce que** la composition comprend en outre une ou plusieurs amidoamines définies par la formule (III) 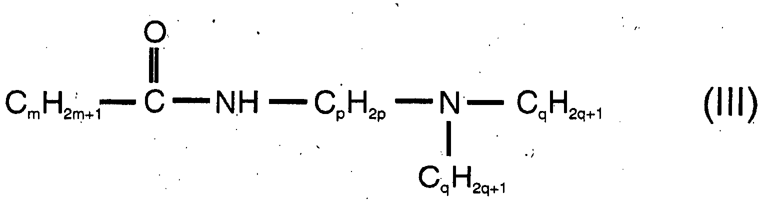 dans laquelle m est un entier dans la plage de 11 à 23, p est un entier dans la plage de 1 à 4 et q est un entier dans la plage de 1 à 4.

6. Composition selon la revendication 5 **caractérisée en ce que** les une ou plusieurs amidoamines sont choisies parmi les stéaramidopropyldiméthylamine, stéaramidopropyldiéthylamine, stéaramidoéthyldiéthylamine, stéaramidoéthyldiméthylamine, palmitamidopropyldiméthylamine, palmitamidopropyldiéthylamine, palmitamidoéthyldiéthylamine, palmitamidoéthyldiméthylamine, béhénamidopropyldiméthylamine, béhénamidopropyldiéthylamine, béhénamidoéthyldiéthylamine, béhénamidoéthyldiméthylamine, arachidamidopropyldiméthylamine, arachidamidopropyldiéthylamine, arachidamidoéthyldiéthylamine, arachidamidoéthyldiméthylamine et des mélanges de celles-ci.

7. Composition selon l'une quelconque des revendications 5 à 6 **caractérisée en ce que** la concentration des une ou plusieurs amidoamines au total est dans la plage de 0,1 % à 5 %, de préférence 0,25 % à 4 % et plus préférablement 0,4 % à 3 % en poids calculée par rapport à la composition totale.

8. Composition selon l'une quelconque des revendications 1 à 7 **caractérisée en ce que** la composition comprend en outre un ou plusieurs alcools gras de formule R₅-OH dans laquelle R₅ représente un groupe hydrocarboné saturé ou insaturé, linéaire ou ramifié, dans laquelle le groupe hydrocarboné comprend 8 à 30 atomes de carbone.

9. Composition selon l'une quelconque des revendications 1 à 8 **caractérisée en ce que** la composition comprend en outre un ou plusieurs sels d'ammonium quaternaire définis par la formule (IV) 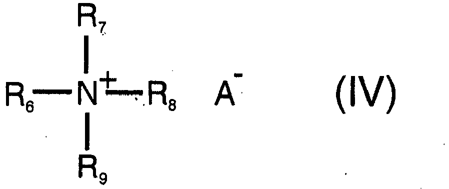
dans laquelle R₆ et R₇ représentent un atome d'hydrogène ou une chaîne alkyle de 1 à 4 atomes de carbone qui sont facultativement substitués par un ou plusieurs groupes hydroxyle,
et dans laquelle R₈ représente une chaîne alkyle saturée ou insaturée, ramifiée ou non ramifiée de 8 à 24 atomes de carbone,
ou R₈ représente un groupe R₁₀CON(CH₂)_{y}, où R₁₀ est une chaîne alkyle saturée ou insaturée, ramifiée ou non ramifiée avec 7 à 21 atomes de carbone et y est un entier dans la plage de 1 à 4,
ou R₈ représente un groupe R₁₁COO (CH₂)_{z}, où R₁₁ représente une chaîne alkyle saturée ou insaturée, ramifiée ou non ramifiée avec 7 à 21 atomes de carbone et z est un entier dans la plage de 1 à 4,
et dans laquelle R₉ représente un atome d'hydrogène ou une chaîne alkyle saturée ou insaturée, ramifiée ou non ramifiée de 8 à 24 atomes de carbone,
ou R₉ représente un groupe R₁₀CON(CH₂)_{y} ou R₁₁COO (CH₂)_{z}, où R₁₀, R₁₁, y et z ont la même' définition que celle décrite ci-dessus, et
A est un anion, de préférence chlorure ou bromure.

10. Composition selon l'une quelconque des revendications 1 à 9 **caractérisée en ce que** la composition comprend en outre de l'eau à une concentration supérieure à 80 % en poids calculée par rapport au poids total de la composition.

11. Composition selon l'une quelconque des revendications 1 à 10 **caractérisée en ce que** la composition est exempte d'huiles de silicone.

12. Composition selon l'une quelconque des revendications 1 à 11 **caractérisée en ce que** la composition est exempte de cellulose contenant des polymères ioniques ou non ioniques.

13. Composition selon l'une quelconque des revendications 1 à 12 **caractérisée en ce que** la composition est exempte de polyquaternium ou de polymères de quaternium.

14. Composition selon l'une quelconque des revendications 1 à 13 **caractérisée en ce que** la composition comprend en outre un ou plusieurs tensioactifs cationiques, anioniques, amphotères et/ou non ioniques.

15. Procédé de conditionnement des cheveux humains, comprenant les étapes d'application de la composition selon l'une quelconque des revendications 1 à 14 sur les cheveux et facultativement rinçage de la composition après son application dans un délai de 1 à 60 minutes.
